# EUROPEAN PATENT APPLICATION

(11) **EP 4 516 901 A1**
(43) Date of publication of application: **05.03.2025**
(21) Application number: 23194740.9
(22) Date of filing: 31.08.2023
(51) Int. Cl.: C12N 15/10, C12N 9/14, C12N 15/70, C12Q 1/6897, C40B 40/02, C40B 40/08

(54) **A METHOD FOR SCREENING OF HYDROLYTIC ENZYMES**

(71) Applicant: Vilnius University, 01513 Vilnius (LT)
(72) Inventor: Urbeliene, Nina, Vilnius (LT); Meskys, Rolandas, Vilnius (LT)
(74) Representative: Petniunaite, Jurga

(57) **Abstract**

A process for identifying biocatalyst having a specified activity of interest, comprises (i) generating one or more expression libraries derived from nucleic acid; and (ii) screening said libraries utilizing fluorescent auxotrophic bacterial cells and a fluorescence detection to identify said biocatalyst. This is a process having a specified activity of interest by (i) generating one or more expression libraries derived from nucleic acid; (ii) exposing said libraries to a particular substrate or substrates of interest; (iii) screening said exposed libraries utilizing a fluorescence activated cell sorter to identify clones which react with the substrate or substrates. Process for identifying clones having a specified activity of interest by (i) generating expression libraries derived from nucleic acid directly or indirectly isolated from the environment; and (ii) screening exposed libraries utilizing an assay requiring co-encapsulation, a binding event or the covalent modification of a target, and a fluorescence detection to identify positive clones.

## Description

### FIELD OF THE INVENTION

The present invention relates generally to the identification of hydrolytic enzymes and particularly to methods for recovering such enzymes by fluorescent auxotrophic bacterial cells and a fluorescence detection.

### BACKGROUND OF THE INVENTION

The selection method based on auxotrophy allows for the identification and isolation of cells that have acquired the desired enzyme or metabolic pathway. It provides a way to screen a large library of genetic material and select for the functional expression of the enzyme in a specific cellular context. In the scientific literature techniques for using amino acids (leucine, histidine, lysine, tryptophan, arginine, sulphur amino acids), nucleosides/nucleotides (uridine, thymidine, ATP, NADH, NAD⁺, NADP⁺) intermediate metabolite (acetyl-CoA, phosphorylated sugars) auxotrophs are described.

An auxotrophic cell is a type of cell that has lost the ability to synthesize or obtain one or more essential nutrients required for its growth and survival. The term "auxotrophy" is derived from the Greek words "auxein" (to increase) and "tropein" (to nourish), indicating the need for external supplementation to support the growth of these cells. For example, uridine auxotrophic cells mean that the genes involved in uridine biosynthesis are disrupted by knocking out. With the result that cells can only divide in media containing uridine source. Therefore, cells with uridine auxotrophy can be used uridine or their analogues as a selective marker to screen and identify cells that have successfully incorporated and expressed the desired genes.

Auxotrophic cells are compatible with a high throughput selection of enzyme activity. A high throughput selection method refers to a process or technique that allows for the rapid screening or selection of a large number of samples or candidates in a relatively short period of time. The screening or selection in the liquid medium can be performed as micro-wells or microfluidics-based technology. It means, that single cells are cultivated in the microwells plates or encapsulated in micro-droplets.

Overall, microfluidics technology offers a versatile and powerful platform for a wide range of applications, including medical diagnostics, drug discovery, single-cell analysis, and environmental monitoring. Its ability to manipulate small volumes of fluids and integrate various analytical processes onto a single chip has the potential to revolutionize research, analysis, and automation in numerous fields.

For the rapid detection of cells division process, often the florescent substrates or fluorescent proteins are use. Fluorescence substrates are molecules that can be used to generate fluorescent signals upon interaction with specific enzymes or biological processes. These substrates typically contain a fluorophore that emits light upon excitation, and their fluorescence intensity can be measured to quantify the activity or presence of the enzyme or process of interest. The choice of substrate depends on the specific application and the target enzyme or process being studied, for examples: i) Protease substrates typically consist of a peptide sequence that can be recognized and cleaved by the protease, resulting in a change in the fluorescence properties of the attached fluorophore; ii) phosphatase often contain a phosphate group attached to a fluorophore, and upon dephosphorylation, the fluorescence intensity changes; iii) Kinase substrates are designed to be phosphorylated by specific kinases, resulting in a change in their fluorescence properties; iiii) Esterase substrates: usually contain an ester linkage attached to a fluorophore, and upon esterase activity, the ester bond is cleaved, leading to a change in fluorescence intensity.

Fluorescent proteins (FPs) are a class of proteins that can absorb and emit light at specific wavelengths, resulting in fluorescence. They are widely used as molecular tags and probes in biological research due to their ability to visualize and track specific molecules or processes within living cells and organisms. Fluorescent proteins can be genetically encoded, allowing them to be expressed within living cells or organisms by incorporating the gene encoding the fluorescent protein into their genome. This genetic encoding enables researchers to target and visualize specific proteins, organelles, or cellular processes by fusing the fluorescent protein gene with the gene of interest. Green fluorescent protein (GFP) is a widely used tool in biotechnology due to its unique ability to emit green fluorescence when exposed to specific wavelengths of light. GFP was originally isolated from the jellyfish *Aequorea victoria,* and its fluorescence property has made it a valuable asset in various applications: GFP can be fused with a target protein of interest to visualize its localization within living cells; as a reporter gene to monitor gene expression levels and promoter activity; in studies of protein-protein interactions and protein complex formation and etc. Beyond GFP, a wide variety of fluorescent proteins with different spectral properties (e.g., blue, cyan, yellow, and red) have been discovered and engineered. This allows for the simultaneous visualization of multiple targets using different coloured fluorescent proteins.

Scheele, R. and et. al. published one of the example of kinases activity detection in living cells by using GFP and droplet-based screening: kinase activity was detected by measuring of GFP fluorescent signal. After phosphorylation of GFP, it became resistant for chymotrypsin digestion. If kinase was inactive, the fluorescent signal was being lost.

The examples of selection based auxotrophy are described in the patent application WO2020227637A1 "Auxotrophic selection methods" and EP0972838B1 "Escherichia coli host/vector system based on antibiotic-free selection by complementation of an auxotrophy". The patent document WO2020227637A1 disclosure provides methods and compositions for generating populations of auxotrophic cells and populations of differentiated cells and selecting populations of transfected cells using split auxotrophy. EP0972838B1 invention concerns new prokaryotic expression systems which enable an antibiotic-free selection and their use for the production of recombinant proteins.

The closest analogue to the invention is described by Jay Short and Martin Keller "High throughput screening for novel enzymes" (US006174673B1). This patent document relates generally to the identification of new bioactive molecules and particularly to methods for recovering such molecules by co-encapsulation and fluorescence activated cell sorting (FACS). The described invention adapts traditional eukaryotic flow cytometry cell Sorting Systems to high throughput screening for expression clones in prokaryotes. The fluorescent substrates are used for the sorting. The method described in the present invention differs from that described in that, that for the detection of enzyme activity could be used any non-fluorescent substrate which is compatible with cell survive. The detection of activity is performed by increased florescent signal of reporter protein (for example GFP). The method allows the analysis of enzymes present in living microorganisms as well as purified/extracted enzymes. Additionally, the desired enzyme and reporter fluorescent protein can be located in the separate cells as well as in the different microorganism system (for example, *E. coli* bacteria and yeast, *E. coli* - *Pseudomonas* and etc.). The system contains one type of cells with reporter protein (reporter cells) and other type of cells with secreted enzyme (producer cells). Both type of cells are cultivated in the one space (tube, droplets and etc). Moreover, this method allows to test enzymes, when substrates are located in the extracellular space and cannot entry into the cells. Also, as the method allows the analysis enzymes toxic for the living cells (for example, proteases and nucleases).

### SUMMARY OF THE INVENTION

This invention provides a method of screening of enzymes with uncharacterized activity in the living cells or in the extracellular space. The present invention relates to screening or selection process of large libraries of polypeptides during which polypeptides conversion of the substrate to its corresponding product compounds.

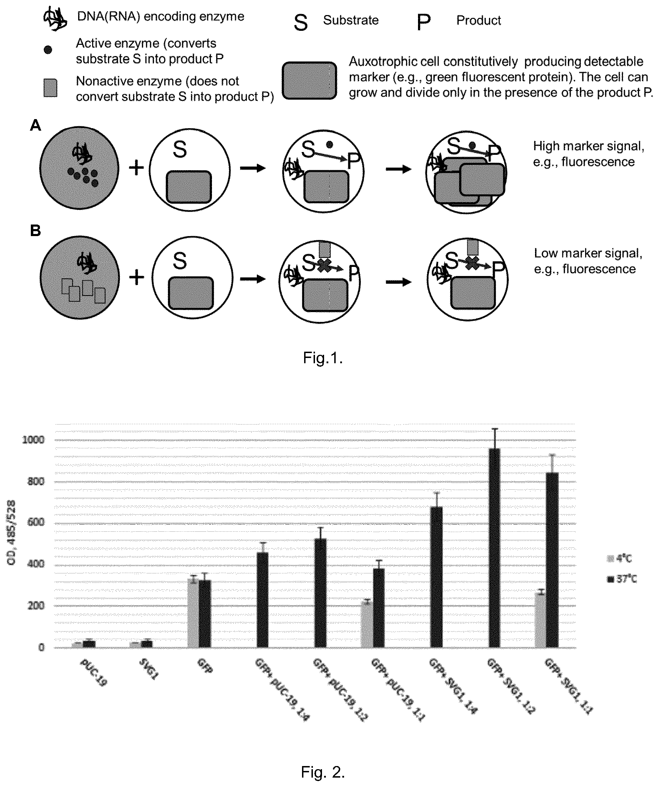

The invention provides a method, which includes the steps (Fig.1):
1. The construction of auxotrophic cells, which are unable to synthesize or acquire the essential nutrient on their own and rely on external supplementation to grow. The cell can grow and divide only in the presence of the product **P.** Auxotrophic cell constitutively producing reporter protein (detectable marker), for example green fluorescent protein.
2. Preparation a selective growth medium that lacks the specific nutrient that the auxotrophic cells require for growth. The selective medium includes an additional supplement substrate **S** that can be converts into product **P** by the functional expression of the desired enzyme. This supplement acts as a selective marker for cells that have acquired the enzyme of interest.
3. Incubation of the auxotrophic cells under appropriate conditions in the selective growth medium. High throughput screening is performed by using micro-well plates or encapsulated microfluidic format. Cells that have successfully acquired and expressed the enzyme of interest will be able to convert the substrate **S** to product **P.** Cells survive, divide and as results bioactive fluorescence signal of refer proteins grow up. Non-transformed cells, cells that have not acquired the desired enzyme or cells together in one space with purified enzymes without desired activity will not be able to grow on the selective medium and the fluorescent signal does not increase.
4. Introduction of producer enzymes can be performed as described below:
   a) a DNA library containing a plurality of genes, wherein the DNA gene codes producer enzyme is introduced into the auxotrophic cells contained reporter fluorescent protein. This can be done using various techniques such as transformation, transfection, or viral transduction, depending on the cell type and organism;
   b) the potential uncharacterized purified enzyme is introduced into the extracellular space through a separate channel;
   c) a DNA library containing a plurality of genes, wherein the DNA gene codes producer enzyme into the separate cells without reporter fluorescent protein. This can be done using various techniques such as transformation, transfection, or viral transduction, depending on the cell type and organism. The auxotrophic cells contained reporter fluorescent protein and cells with potential enzyme are cultivated in the one isolated space-in the one micro-well or emulsion drop.
5. The cells are detected by fluorescent signal intensity by using FACS (Fluorescence-activated Cell Sorting) system or fluorimeter.
6. Analysis of the surviving colonies to confirm the presence of the desired genetic material. The identified colonies can then be further expanded and characterized.

### BRIEF DESCRIPTION OF FIGURES

The features of the invention, which is new and non-obvious, are given in the Claims. However, the invention may be best understood from the following detailed description of the invention, in which, without limiting the scope of the invention, exemplary embodiments of the invention are given in conjunction with the accompanying drawings, where:
**Fig. 1** depicts the principle schema of provided method: **A** - Cells that have successfully acquired and expressed the enzyme of interest will be able to convert the substrate **S** to product **P.** Cells survive, divide and as results fluorescence signal of refer proteins grow up. **B** - Cells that have not acquired and not expressed the enzyme of interest will be not able to convert the substrate **S** to product **P.** Cells not divide and as results fluorescence signal of refer proteins lost.
**Fig. 2****.** depicts the cells growing represented by a column chart. The fluorescence signal of GFP was measured after 18 hours of *E*. *coli* cells incubation at 37 °C. Parameters of the measuring were as follows: Excitation-485nm, Emission-528 nm and gain equal 50. Marking meanings: "Puc19"-cells transformed with empty vector pUC19; "SVG1"-cells transformed with plasmid pUC19 harbouring esterase SVG1 gene; "GFP"-cells transformed with pSTV28 harbouring sfGFP gene; "GFP+ pUC-19, 1:4", "GFP+ pUC-19, 1:2", "GFP+ pUC-19, 1:1"-cells transformed with empty pUC19 vector and pSTV28 harbouring sfGFP gene "GFP+ SVG1, 1:4", "GFP+ SVG1, 1:2"; "GFP+ SVG1, 1:1" cells transformed with plasmid pUC19 harbouring esterase SVG1 gene and pSTV28 harbouring sfGFP gene; 1:4, 1:2 and 1:1 refers to the ratio of GFP:SVG1 cells. The bioactive fluorescence level of the initial reaction mixtures was measured after the samples were stored at 4 °C.

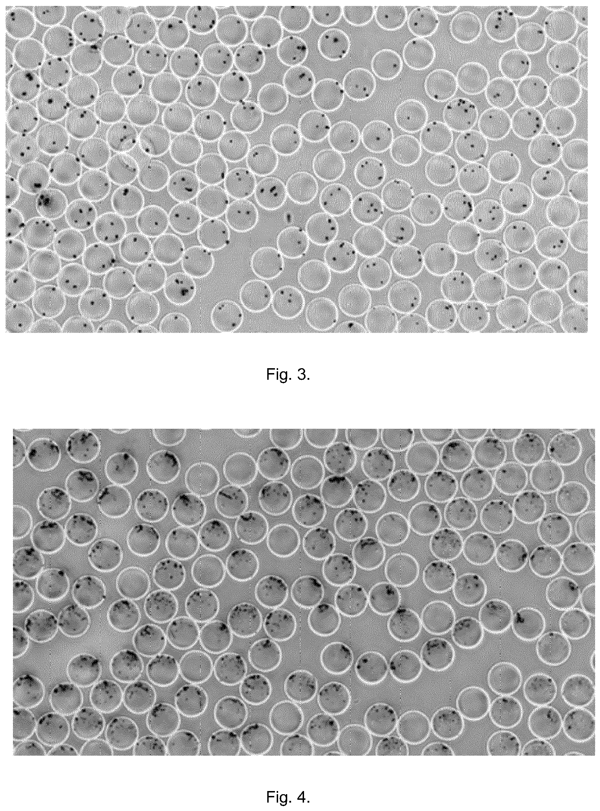

**Fig. 3****.** depicts the fluorescent microscopy image of encapsulated bacteria in micro-droplets. The black spots in the emulsion drops refers to cells with sfGFP protein. The detection of fluorescent was performed at 488nm wavelength.
**Fig. 4****.** depicts the fluorescent microscopy image of bacteria in micro-droplets after 18 h incubation at 37C. The black spots in the emulsion drops refers to cells with sfGFP protein. Compared to the initial encapsulation image (Figure 3), an increasing number of spots is observed. The detection of fluorescent was performed at 488nm wavelength.

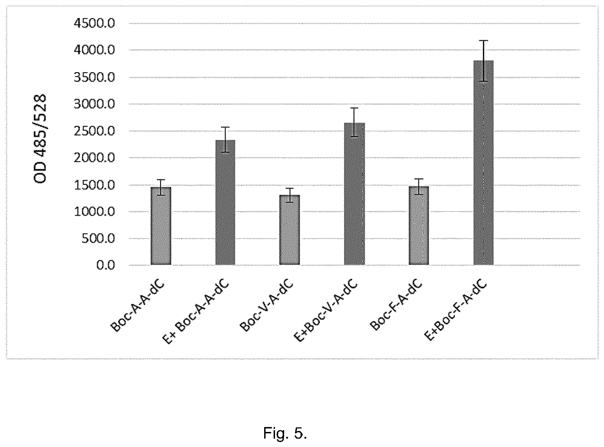

**Fig. 5****.** depicts the cells growing represented by a column chart. The fluorescence signal of GFP was measured after 20 hours of *E*. *coli* cells incubation at 37 °C. Parameters of the measuring were as follows: Excitation-485nm, Emission-528 nm and gain equal 50. Marking meanings: Boc-A-A-dC,Boc-A-V-dC, Boc-A-F-dC - control samples with cytidine derivatives and without RNAse A; E+Boc-A-A-dC, E+Boc-A-V-dC, , E+Boc-A-F-dC - samples with cytidine derivatives ant with RnaseA.

### DETAILED DESCRIPTION OF THE INVENTION

In the present invention screening is based on auxotrophic cells and fluorescent signal of reporter protein, generated by host cells. For example, by using uridine auxotrophic *E. coli* cells and selecting for the ability to grow in the absence of uridine, this method enables the identification and isolation of cells that have acquired the capacity to convert modified uridine analogue to uridine or synthesize uridine or express an enzyme involved in uridine biosynthesis.

Disclosed is a process for identifying biocatalyst having a specified activity of interest, which process comprises (i) generating one or more expression libraries derived from nucleic acid; and (ii) screening said libraries utilizing fluorescent auxotrophic bacterial cells and a fluorescence detection to identify said biocatalyst. More particularly, this is a process for identifying biocatalyst having a specified activity of interest by (i) generating one or more expression libraries derived from nucleic acid; (ii) exposing said libraries to a particular substrate or substrates of interest; and (iii) screening said exposed libraries utilizing a fluorescence activated cell sorter to identify clones which react with the substrate or substrates. Also, provided is a process for identifying clones having a specified activity of interest by (i) generating one or more expression libraries derived from nucleic acid directly or indirectly isolated from the environment; and (ii) screening said exposed libraries utilizing an assay requiring co-encapsulation, a binding event or the covalent modification of a target, and a fluorescence detection to identify positive clones.

The present invention may be employed for example, to identify enzymes with desired activity of regio/enantio-selective lipases, esterases, amidohydrolases (for example, amidases, proteases), glycosidases, glycosyl transferases, acylases, lyases, nucleases, phosphatases. In the present invention screening is based on auxotrophic cells allowed to select:
i) Purified/extracted toxic enzyme in extracellular space;
ii) Secreted enzyme generated in the other host cells separate from cells with reporter protein;
iii) Intracellular enzyme produced in the reporter cells.

The methods of the invention allow identification and characterization of enzyme with previously unknown or undefined enzymatic activity. The enzyme can by selected from:
i) Gene libraries or metagenomic libraries generated from one or more cultivated or uncultivated microorganisms are screened for an activity of interest. Libraries are generated, clones are either exposed to the substrate or substrate(s) of interest, and positive clones are identified and isolated via fluorescence signal of reporter cells;
ii) Gene mutant's libraries generated by mutagenesis of wild type enzyme gene.
iii) Polypeptide libraries.

In the present invention desired substrate can be administered to the cells:
i) The substrates can be intracellular, which can be transporter to the cells by cells wall channels by active transporter or by inactive diffusion;
ii) The substrates can be extracellular, for example DNA, RNA, and other bulkiness organic non-transported molecules;

In the present invention screening of enzyme activity can be performed:
i) in Multi-well plates,
ii) in microdroplets generated by emulsification or by Micro-fluids system.

The term "Producer enzyme" as used therein, unless otherwise specified, refers to an enzyme which converts substrate (**S**) to product (**P**).

The term "Reporter cells" or the term "fluorescent marker" as used therein, unless otherwise specified, refers to auxotrophic cells, which produces fluorescent protein, called reporter protein.

The term "bioactive fluorescence" as used therein, unless otherwise specified, refers to a fluorescence of the reporter cells.

The term Substrate (**S**) as used therein, unless otherwise specified, refers to an organic compound which is not metabolised in the auxotrophic cells used for enzyme screening. The screened producer enzyme converts substrate (**S**) to product (**P**). Product **P** is essential for growth and survival of reporter cells.

The term Product (**P**) as used therein, unless otherwise specified, refers to essential nutrients required for growth and survival of auxotrophic cells. The product (**P**) is produced from substrate S by using producer enzyme.

The term selective medium as used therein, unless otherwise specified, refers to a mineral medium that lacks the specific nutrient that the auxotrophic cells require for growth. The selective medium includes an additional supplement substrate **S** that can be converts into product **P** by the functional expression of the desired enzyme.

The term "Polypeptide library/libraries" as used therein, unless otherwise specified, refers to a pool of enzymes which activity are previously uncharacterised towards the substrate.

The term "Screening of enzymes" refers to evaluation of every protein for the desired property, while "selection of enzymes" automatically eliminates non-functional variants. Selection or screening of enzymes can be performed from enzyme libraries *in vivo* or *in vitro.* The term "enzyme library" refers to a set of proteins or genes inserted into expression vectors or cells producing the enzymes. The enzyme library can be a metagenomic library, genomic library or a mutant library. The metagenomic library can be constructed from various sources of environmental sample, such as soil, water, sludge, and etc.

The term "Host cell" as used therein, unless otherwise specified, refers to the cells, transformed by a DNA vector harbouring the nucleic acid/DNA gene potentially coded producer enzymes.

The term "fluorescent analyser" as used therein, unless otherwise specified, refers to ay device, which can be used for detection and measurement of a fluorescence.

### EMBODIMENTS OF THE INVENTION

The following is information on specific examples describing implementation of the invention. Embodiments of the invention are provided for illustration; they do not limit the scope of the invention.

Examples of implementation of the invention:

### Example 1. Screening of secreted lipases/esterases using GFP fluorescent signal and uridine auxotrophic E. coli cells harbouring hydrolase gene in the selected liquid media

*E. coli* DH10BΔ*pyrFEC* competent cells were transformed with compatible (p15 origin) plasmids harbouring superfold green fluorescent protein (sfGFP), for example pSTV28 vector with sfGFP gene. The other *E*. *coli* DH10BΔ*pyrFEC* competent cells were transformed with plasmids harbouring secreted esterase pUC19-SVG1. As a negative control, the PUC19 vector, which lacks the esterase gene was used. After transformation cells were cultivated in the selective M9 medium containing 20 µg/ml chloramphenicol, 100 µg/ml ampicillin, and 20 µg/ml uridine analogue - 2',3',5'-*O*-acetyluridine substrate transported into the cells. The secreted esterase deacylated 2',3',5'-*O*-acetyluridine into uridine in the growing media, upon which the growth phenotype of uridine auxotrophic cells with sfGFP is restored. As a result, if the clone harbours a gene encoding the active secreted lipase/esterase, the GFP fluorescent signal growing is observed by cultivation (fig. 2). The fluorescence signal of GFP was measured using a plate reader spectrophotometer in black 96 well plates. Parameters of the measuring were as follows: Excitation - 485nm, Emission - 528 nm and gain equal 50. Parameters of the measuring were as follows: Excitation-485nm, Emission-528 nm and gain equal 50.

### Example 2. Screening of secreted lipases/esterases using GFP fluorescent signal and uridine auxotrophic E. coli cells harbouring hydrolase gene in micro-droplets

Uridine auxotrophic strain *E*. *coli* DH10BΔ*pyrFEC* competent cells were transformed with plasmids harbouring super-fold green fluorescent protein (sfGFP), for example pSTV28 vector (p15 origin) with sfGFP gene. The other *E*. *coli* DH10BΔ*pyrFEC* competent cells were transformed with plasmids harbouring secreted esterase pUC19-SVG1 and co-transformed with compatible plasmids harbouring reporter protein RFP1 in pSTV28 vector. The reporter RFP1 permit observed division of producer's cells with hydrolase gene. As a negative control, the PUC19 vector, without the esterase gene, was used. After transformation cells were cultivated in SOC media 45 min. After that cells were collected by centrifugation and transferred into the selective M9 medium containing 20 µg/ml chloramphenicol, 100 µg/ml ampicillin, and 20 µg/ml uridine analogue - 2',3',5'-*O*-acetyluridine substrate transported into the cells. Cells were incubated in the M9 medium 3-4 h until exponential growth phase. Then the single cells (λ=5 Poisson distribution) were encapsulated into microfluidic droplets (droplet volume -22,5pL) by using ONYX droplet generator. After encapsulation the droplets with cells were incubated 18-24 h at 37°C, without shaking. When secreted esterase deacylated 2',3',5'-*O*-acetyluridine into uridine, the growth phenotype of uridine auxotrophic cells with sfGFP is restored. As a result, if the clone harbours a gene encoding the active secreted lipase/esterase, the growing of GFP fluorescent signal was observed. The fluorescence signal did not increase in the negative control samples (with empty pUC19 vector). Bacteria growth in droplets were monitored by florescent microscope after 0h; 24h; 96h incubation time. The sorting of cells by intensity of fluorescence was performed by using the STYX HTS platform.

### Example 3. Screening of metagenomic amidohydrolases using GFP fluorescent signal and uridine auxotrophic E. coli cells harbouring hydrolase gene in micro-droplets

Uridine auxotrophic strain *E*. *coli* DH10BΔ*pyrFEC* competent cells were transformed with metagenomic library and with compatible plasmids harbouring superfold green fluorescent protein (sfGFP), for example pSTV28 vector (p15 origin) with sfGFP gene. As a negative control the PUC19 vector without the hydrolase gene, was used. After transformation cells were cultivated in SOC media 45 min. After that cells were collected by centrifugation and transferred into the selective M9 medium containing 20 µg/ml chloramphenicol, 100 µg/ml ampicillin, and 20 µg/ml uridine analogue-*N*⁴-benzoil-2'-deoxycytidine substrate transported into the cells. Cells were incubated in the M9 medium 3-4 h until exponential growth phase. Then the single cells (λ=5 Poisson distribution) were encapsulated into microfluidic droplets (droplet volume -22,5pL) by using ONYX droplet generator. The droplets with cells were incubated 18-24 h at 37°C, without shaking. When amidohydrolase or cytidine deaminase from metagenomic library converted *N*⁴-benzoil-2'-deoxycytidine into 2'-deoxycytidine/2'-deoxyuridine, the growth phenotype of uridine auxotrophic cells is restored. As a result, if the clone harbours a gene encoding the active hydrolase, the growing of GFP fluorescent signal was observed. The fluorescence signal did not increase in the negative control samples (with empty pUC19 vector). Bacteria growth in droplets were monitored by florescent microscope after 0h, 18-24h, 48h incubation time. The sorting of cells by intensity of fluorescence was performed by using the STYX HTS platform (fig. 3 and fig. 4)

### Example 4. Screening of active mutants of amidohydrolases from mutant library by using GFP fluorescent signal and uridine auxotrophic E. coli cells harbouring hydrolase gene

Uridine auxotrophic strain *E. coli* DH10BΔ*pyrFEC* competent cells were transformed with set of 24 mutants of cytidine deaminase CDA_F14 and with compatible plasmids harbouring superfold green fluorescent protein (sfGFP), for example pSTV28 vector (p15 origin) with sfGFP gene. After transformation cells were cultivated in SOC media 45 min. After that cells were collected by centrifugation and transferred into the selective M9 medium containing 20 µg/ml chloramphenicol, 100 µg/ml ampicillin, and 20 µg/ml uridine analogue-*N*⁴-benzoil-2'-deoxycytidine substrate transported into the cells. Cells were incubated in the M9 medium 3-4 h until exponential growth phase. Then the single cells (λ=5 Poisson distribution) were encapsulated into microfluidic droplets (droplet volume -22,5pL) by using ONYX droplet generator. The droplets with cells were incubated 18-24 h at 37°C, without shaking. When amidohydralase or cytidine deaminase from metagenomic library converted *N*⁴-benzoil-2'-deoxycytidine into 2'-deoxyuridine, the growth phenotype of uridine auxotrophic cells is restored. As a result, if the clone harbours a gene encoding the active hydrolase, the growing of GFP fluorescent signal was observed. Seven active enzymes were selected from the 24 mutants set. Bacteria growth in droplets were monitored by florescent microscope after 0h and 18-24h incubation time. The sorting of cells by intensity of fluorescence was performed by using the STYX HTS platform.

### Example 5. Screening of RNA degrading enzymes using GFP fluorescent signal and uridine auxotrophic E. coli in the selected liquid media

Uridine auxotrophic strain *E. coli* DH10BΔ*pyrFEC* cells containing plasmids with super-fold green fluorescent protein (pSTV28_sfGFP) were cultivated until exponential grow phase (optical density OD₆₀₀=0.5-0.6) into LB medium at 37°C. After that cells were collected by centrifugation and transferred into the selective M9 medium containing 20 µg/ml chloramphenicol and 0.35 mg/ml RNA as uridine source and 18 U/ml FastAP thermosensitive alkaline phosphatase. 0.18 mg/ml Rnase A or mix of 5 kU/ml universal nuclease and 0.05 U/ml phosphodiesterase I from *Crotalus adamanteus* venom was added into the samples. A mili-Q water was added into the control samples instead of the nucleases. Then cells were incubated 18-24 h in the sterile 96-well plate at 37°C, under aeration. The fluorescence signal of GFP was measured using a plate reader spectrophotometer in black 96 well plate. Parameters of the measuring were as follows: Excitation-485nm, Emission-528 nm and gain equal 50. RNA is a substrate, which is not transported into the cells. The nucleoside monophosphates are produced after digestion RNA by RNAse A or universal nuclease and phosphodiesterase I from *Crotalus adamanteus* venom. FastAP thermosensitive alkaline phosphatase dephosphorylates nucleoside monophosphates into nucleosides. After that only the produced nucleosides are transported to the cells. As a results uridine auxotrophic cells can grow in the selective medium due to the formation of uridine nucleoside from RNA.

### Example 6. Screening of DNA degrading enzymes using GFP fluorescent signal and uridine auxotrophic E. coli in the selected liquid media

Uridine auxotrophic strain *E. coli* DH10BΔ*pyrFEC* cells containing plasmids with super-fold green fluorescent protein (pSTV28_sfGFP) were cultivated until exponential grow phase (optical density OD₆₀₀=0.5-0.6) in the LB medium at 37°C. After that cells were collected by centrifugation and transferred into the selective M9 medium containing 20 µg/ml chloramphenicol, 2 mg/ml *Calf thymus* DNA as uridine source and 18 U/ml FastAP thermosensitive alkaline phosphatase. 0.002 U/ml Exonuclease III or 5 kU/ml universal nuclease was added into the samples. A mili-Q water was added into the control samples instead of the nucleases. Then cells were incubated 18-24 h in the sterile 96-well plate at 37°C, under aeration. The fluorescence signal of GFP was measured using a plate reader spectrophotometer in black 96 well plate. Parameters of the measuring were as follows: Excitation-485nm, Emission-528 nm and gain equal 50. DNA is a substrate, which is not transported into the cells. The nucleoside monophosphates are produced after digestion DNA by Exonuclease III or universal nuclease. FastAP thermosensitive alkaline phosphatase dephosphorylates deoxynucleoside monophosphates into deoxynucleosides. After that only the produced deoxynucleosides are transported to the cells. As a results uridine auxotrophic cells can grow in the selective medium due to the formation of uridine from DNA.

### Example 7. Screening of peptides degrading enzymes using GFP fluorescent signal and uridine auxotrophic E. coli in the selected liquid media

Uridine auxotrophic strain *E. coli* DH10BΔ*pyrFEC* cells containing plasmids with super-fold green fluorescent protein (pSTV28_sfGFP) were cultivated until exponential grow phase (optical density OD₆₀₀=0.5-0.6) in the LB medium at 37°C. After that cells were collected by centrifugation and transferred into the selective M9 medium containing 20 µg/ml chloramphenicol and 0.5 mM deoxycytidine derivative N4-Ala-β-alaninoyl-Boc-2'-deoxycitidine (Boc-β-Ala-Ala-dC) or N4-Val-β-alaninoyl-Boc-2'-deoxycitidine (Boc-β-Ala-Val-dC) or N4-Phe-β-alaninoyl-Boc-2'-deoxycitidine (Boc-β-Ala-Phe-dC) as uridine source. Boc-β-Ala-alaAla-dC, Boc-β-Ala-Val-dC and Boc-β-Ala-Phe-dC were prepared as follows.

The corresponding dipeptide (β-Ala-Ala - 2.04 mmol, β-Ala-Val - 2.58 mmol and β-Ala-Phe - 2.08 mmol) was dissolved in 10 ml of a 1:1 mixture of water and 1,4-dioxane. The mixture was stirred with a magnetic stirrer and 3.9 ml NaOH (1 M) was added slowly. After the dipeptide was dissolved, the reaction mixture was cooled in an ice bath, 1.5 mol eq. of di-tert-butyldicarbonate (Boc₂O) and stirred at 50°C for 24 hours. The reaction mixtures were evaporated with a rotary evaporator and acidified with 10% citric acid solution to pH 4. Extracted four times with 50 ml, 50 ml, 40 ml and 25 ml of ethyl acetate. Fractions containing the product were dried with Na₂SO₄, filtered and evaporated on a rotary evaporator. The synthesized dipeptides blocked by the Boc protective group were dissolved in 15 ml of ethyl acetate and mixed with a magnetic stirrer. 1.5 mol equiv was added. (0.445 g) of N-hydroxysuccinimide (NHS) and 1.5 mol. eq. (0.800 g) of N,N'-dicyclohexylcarbodiimide (DCC). The reactions were carried out for 24 h. at room temperature. The precipitate formed was filtered, and the ethyl acetate was evaporated using a rotary evaporator. Activated Boc protected dipeptides were dissolved in 7 ml of dimethylformamide (DMF) and 1.5 mol. eq. dCyd. The reaction was carried out for 7 days at room temperature. After the reaction, the solvent was evaporated with a rotary evaporator. N4-Acyl-Boc-2'-deoxycytidines were purified by column chromatography using a column filled with 60 ml of silica gel suspended in CHCl₃. Purification started with CHCl₃, further purification with mixtures of CHCl₃ and CH₃OH, gradually increasing the methanol concentration to 10%. Fractions in which the pure product was detected were evaporated using a rotary evaporator. N4-Ala-β-alaninoyl-Boc-2'-deoxycitidine. (Obtained 300 mg (469,2 g/mol). Yield 32%. MS (ESI+): m/z 470,2 [M+H]+, 468.2 [M-H]-.1H NMR (DMSO-d6, 400 MHz): δ = 1.06-1.16 (m, 3H, CH3); 1.36 (s, 9H, 3CH3); 2.00 (dq, J = 12.5, 6.2 Hz, 1H, CH2); 2.19-2.34 (m, 1H, CH2); 2.48-2.57 (m, 2H, CH2); 3.20-3,36 (m, 1H, CH2); 3.43 (t, J = 6,4 Hz, 1H, CH2); 3.55-3.63 (m, 2H, CH2); 3.79-3.95 (m, 2H, 2CH); 4.21 (s, 1H, CH); 5.06 (s, 1H, OH); 5.27 (m, 1H, OH); 6.11 (t, J = 6.3 Hz, 1H, CH); 6.87 (d, J = 7.6 Hz, 1H, NH); 7.21 (d, J = 7.5 Hz, CH=CH); 7.81 (t, J = 5.7 Hz, 1H, NH); 8.34 (d, J = 7.5 Hz, CH=CH); 10.90 (s, 1H, NH). 13C NMR (DMSO-d6, 100 MHz): δ = 18.89; 28.64; 34.82; 36.88; 41.26; 51.95; 61.43; 70.41; 79.63; 86.45; 88.28; 95.82; 145.39; 154.91; 155.42; 162.66; 172.24; 172,64.) N4-Val-β-alaninoyl-Boc-2'-deoxycitidine (Obtained 413 mg (497.6 g/mol). Yield 32%. MS (ESI+): m/z 398.55 [M+H]+, 396.55 [M-H]-. 1H NMR (DMSO-d6, 400 MHz): δ = 1.28 (s, 9H, 3CH3); 1.96-2.05 (m, 1H, CH2); 2.21-2.38 (m, 1H, CH2); 2.58 (dd, J = 12.0, 5.3 Hz, 2H, 2CH2); 2.65-2.78 (m, 1H, CH2); 2.91 (dd, J = 13.7, 4.5 Hz, 1H, CH2); 3.17 (d, J = 5.1 Hz, 1H, CH2); 3.24-3.41 (m, 1H, CH2); 3.57 (d, J = 12.1 Hz, 1H, CH2); 3.63 (dt, J = 12.3, 4.6 Hz, 1H, CH2); 3.86 (q, J = 3.7 Hz, 1H, CH); 4.10 (dq, J = 9.5, 4.8 Hz, 1H, CH); 4.22 (dd, J = 6.0, 3.5 Hz, 1H, CH); 5.02 (t, J = 5.2 Hz, 1H, OH); 5.28 (d, J = 4.2 Hz, 1H, OH); 6.11 (t, J = 6.3 Hz, 1H, CH); 6.87 (d, J = 8.6 Hz, 1H, NH); 7.16-7.19 (m, 1H, CH=CH); 7.20-7.29 (m, 5H, 5CH); 7.97 (t, J = 5.8 Hz, 1H, NH); 8.34 (d, J = 7.5 Hz, 1H, CH=CH); 10.92 (s, 1H, NH). 13C NMR (DMSO-d6, 100 MHz): δ = 26.46; 32.73; 34.72; 36.02; 39.27; 46.95; 54.02; 59.30; 68.28; 76.32; 84.51; 86.26; 93.71; 116.41; 124.48; 126.34; 127.50; 136.51; 143.29; 152.81; 153.49; 160.56; 169.96; 170.50). N4-Phe-β-alaninoyl-Boc-2'-deoxycitidine (Obtained 900 mg (545.3 g/mol). Yield 83%. MS (ESI+): m/z 546.25 [M+H]+, 544.25 [M-H]-. 1H NMR (DMSO-d6, 400 MHz): δ = 0.79 (t, J = 6.5 Hz, 6H, 2CH3); 1.36 (s, 9H, 3CH3); 1.87 (p, J = 6.8 Hz, 1H, CH); 2.01 (dt, J = 13.0, 6.3 Hz, 1H, CH2); 2.29 (ddd, J = 13.3, 6.1, 3.9 Hz, 1H, CH2); 2.59 (t, J = 3.5 Hz, 2H, 2CH2); 3.22-3.32 (m, 1H, CH2); 3.33-3.42 (m, 1H, CH2); 3.60 (tdd, J = 12.2, 8.4, 3.8 Hz, 2H, 2CH2); 3.67-3.74 (m, 1H, CH); 3.86 (q, J = 3.7 Hz, 1H, CH); 4.22 (dt, J = 6.2, 3,3 Hz, 1H, CH); 5.07 (d, J = 5.2 Hz, 1H, OH); 5.28 (d, J = 4.1 Hz, 1H, OH); 6.11 (t, J = 6.3 Hz, 1H, CH); 6.61 (d, J = 9.0 Hz, 1H, NH); 7.21 (d, J = 7.5 Hz, 1H, CH=CH); 7.91 (t, J = 5.7 Hz, 1H, NH); 8.33 (d, J = 7.6 Hz, 1H, CH=CH); 10.89 (s, 1H, NH). 13C NMR (DMSO-d6, 100 MHz): δ = 18.69; 19.62; 28.63; 30.85; 34.77; 36.79; 41.37; 60.09; 61.41; 70.39; 79.63; 86.60; 88.36; 95.80; 145.38; 154.90; 155.82; 162.65; 171.80; 172.57).

Additionally, 1 mg/ml Proteinase K was added into the samples. A mili-Q water was added into the control samples instead of the proteinase K. Then cells were incubated 20 h in the sterile 96-well plate at 37°C, under aeration. The fluorescence signal of GFP was measured using a plate reader spectrophotometer in black 96 well plate. Parameters of the measuring were as follows: Excitation-485nm, Emission-528 nm and gain equal 50.

### Example 8. Screening of RNA degrading enzymes using GFP fluorescent signal and uridine auxotrophic E. coli in micro-droplets

Uridine auxotrophic strain *E. coli* DH10BΔ*pyrFEC* cells containing plasmids with super-fold green fluorescent protein (pSTV28_sfGFP) were cultivated until exponential grow phase (optical density OD₆₀₀=0.5-0.6) into LB medium at 37°C. After that cells were collected by centrifugation and transferred into the selective M9 medium containing 20 µg/ml chloramphenicol and 0.35 mg/ml RNA as uridine source and 18 U/ml FastAP thermosensitive alkaline phosphatase. Then the single cells (λ=5 Poisson distribution) were encapsulated into microfluidic droplets (droplet volume -22,5pL) by using ONYX droplet generator and microchip with three incoming channels. 0.5 mg/ml Rnase A in 50 mM Tris-HCl, pH8.0, 1 mM MgCl₂ buffer was added into the micro-droplets via a separate channel (separately from cells). A buffer was added into the control samples instead of the nucleases. The prepared droplets with cells were incubated 18 h at 37°C, without shaking. As a result, a division of cells and growing of GFP fluorescent signal was observed in the samples with Rnase A. RNA is a substrate, which is not transported into the cells. The nucleoside monophosphates are produced after digestion RNA by RNAse A. FastAP thermosensitive alkaline phosphatase dephosphorylates nucleoside monophosphates into nucleosides. After that only the produced nucleosides are transported to the cells. As a results uridine auxotrophic cells can grow in the selective medium due to the formation of uridine nucleoside from RNA. No cells division was observed, and the fluorescence signal did not increase in the negative control samples without Rnase A. Bacteria growth in droplets were monitored by florescent microscope after 18 h incubation time.

### Literature

Autour, A.; Ryckelynck, M. Ultrahigh-Throughput Improvement and Discovery of Enzymes Using Droplet-Based Microfluidic Screening. Micromachines 2017, 8, 128. https://doi.orp/10.3390/mi8040128.
Chen, J, Wang, Y., Zheng, P., Sun, J. Engineering synthetic auxotrophs for growth-coupled directed protein evolution. Trends Biotechnol. 2022 Jul;40(7):773-776. doi: 10.1016/j.tibtech.2022.01.010. Epub 2022 Feb 12. PMID: 35168803.
Neun, S., Brear, P., Campbell, E. et al. Functional metagenomic screening identifies an unexpected β-glucuronidase. Nat Chem Biol 18, 1096-1103 (2022). https://doi.org/10.1038/s41589-022-01071-x
Scheele, R.A., Lindenburg, L.H., Petek, M. et al. Droplet-based screening of phosphate transfer catalysis reveals how epistasis shapes MAP kinase interactions with substrates. Nat Commun 13, 844 (2022). https://doi.ora/10.1038/s41467-022-28396-4)
Yuan H, Tu R, Tong X, Lin Y, Zhang Y, Wang Q. Ultrahigh-throughput screening of industrial enzyme-producing strains by droplet-based microfluidic system. J Ind Microbiol Biotechnol. 2022 May 25;49(3):kuac007. doi: 10.1093/jimb/kuac007. PMID: 35259275; PMCID: PMC9142201.
WO2020227637A1
EP0972838B1
US006174673B1

## Claims

1. A method for screening hydrolytic enzymes comprising:
(a) DNA library containing a plurality of genes, wherein the DNA gene codes producer enzyme, which convert the substrate to the product;
(b) Construction of a DNA vector harbouring the nucleic acid/DNA gene coded producer enzyme;
(c) Host cell transformation with the DNA vector from step (b);
(d) Auxotrophic cells with reporter fluorescent protein;
(e) A selective growth medium that lacks the specific nutrient that the auxotrophic cells require for growth and a substrate which can be converted to a product, by producer enzyme;

2. The method according to claim 1, wherein producer enzyme is produced *in vivo* in the one cell with reporter;

3. The method according to claim 1, wherein producer enzyme is produced *in vivo* in the separate cells;

4. The method according to claim 1, providing a DNA library containing a plurality of genes, wherein the DNA for generating the libraries obtained from a metagenomic DNA;

5. The method according to claim 1, providing a DNA library containing a plurality of genes, wherein the DNA for generating the library is obtained from mutants' libraries;

6. The method according to claim 1, wherein screening of enzymes is performed in the micro-droplet with a fluorescent analyser that detects bioactive fluorescence;

7. The method according to claim 1, wherein screening of enzymes is performed in the micro-well plates with a fluorescent analyser that detects bioactive fluorescence;

8. The method according to claim 1, wherein substrate is transported into the cells;

9. The method according to claim 1, wherein substrate is not transported into the cells;

10. The method according to claim 9, wherein the producer enzyme is secreted enzyme of other host cells;

11. The method according to claim 9, wherein the producer enzyme is selected from polypeptide library/libraries;

12. The method of claim 1, wherein the bioactivity is an enzyme is selected from the group consisting region/enantio-selective lipases, esterases, amidohydrolases (amidases, proteases), glycosidases, glycosyl transferases, acylases, lyases, nucleases, phosphatases.
